# EUROPEAN PATENT APPLICATION

(11) **EP 1 086 704 A1**
(43) Date of publication of application: **28.03.2001**
(21) Application number: 99923873.6
(22) Date of filing: 02.06.1999
(51) Int. Cl.: A61K 45/00, A61K 38/17, G01N 31/22

(54) **EOSINOPHIL CHEMOTACTIC FACTOR**

(30) Priority: 03.06.1998 JP 17069898
(71) Applicant: Effector Cell Institute, Tokyo 153-0041 (JP)
(72) Inventor: KANEGASAKI, Shiro, Kawasaki-shi, Kanagawa 214-0032 (JP); MATSUMOTO, Ryoji, Rochester, MN 55906 (US); HIRASHIMA, Mitsuomi, Takamatsu-shi, Kagawa 761-0322 (JP)
(74) Representative: Holdcroft, James Gerald, Dr.
(86) International application number: JP9902952
(87) International publication number: WO9962556

(57) **Abstract**

An eosinophil chemotactic factor which is constantly secreted by a T cell clone STO-2 is partially purified and a specific antibody was generated. Using this antibody, a DNA library derived from the clone was screened to find that ecalectin, a galectin consisting of 323 amino acids has a specific eosinophil chemotactic activity. Use of the galectin makes it possible to provide an agent having an effect of increasing eosinophils in a tissue, and a method for screening compounds inhibiting the effect. Moreover, an agent containing as an effective ingredient a compound that inhibits eosinophil increase in a tissue, is effective as therapeutic, preventive or ameliorative agents for various allergic diseases, such as bronchial asthma, allergic rhinitis and atopic dermatitis, which are typical delayed inflammatory diseases attributed to eosinophils.

## Description

### Technical field

The present invention relates to the field of genetic engineering, specifically DNA encoding a protein comprising an eosinophil chemotactic activity.

### Background Art

It has been revealed that delayed inflammations caused by eosinophils occur in various allergic diseases, for example, bronchial asthma, allergic rhinitis, and atopic dermatitis, as well as in parasitization (Adv. Immunol. 39, 177-253, 1986).

Generally, eosinophils exist as a part of leukocytes in a small amount in circulating blood, however, the number of eosinophils in blood occasionally increase to develop eosinophilia. Eosinophil chemotactic factors induced in a tissue by some cause mediate introduction of eosinophils in the blood into the tissue from blood vessels. Therefore, revealing the factors related to eosinophilia and chemotaxis and controlling their direct or indirect effects may lead to treatment of the above allergic diseases.

As peptidergic factors which mediate migration of eosinophils in *in vitro* experiments and the like, eotaxin, granulocyte-macrophage colony stimulating factor (GM-CSF), interleukin-2 (IL-2), interleukin-3 (IL-3), interleukin-5 (IL-5), interleukin-8 (IL-8), interleukin-16 (IL-16), RANTES, MIP-1α, MCP-2, MCP-3, MCP-4, C3a, and C5a have been reported. Most of them, however, have low levels of activity to specifically migrate eosinophils. Among them, IL-5 and eotaxin are chemotactic factors with a relatively high specificity against eosinophils, however, the chemotactic factor activity of IL-5 is very weak, and eotaxin is produced at quite a low level by T lymphocytes activated by an antigen at allergic inflammation sites (J. Immunol. 160 (1): 60-68, 1998).

### Disclosure of the Invention

An objective of the present invention is to find an eosinophil-specific factor that induces eosinophil chemotaxis, and to develop a system for screening drugs for controlling migration of eosinophils by using this factor.

The present inventors hypothesized the existence of an eosinophil-specific chemotactic factor produced by T lymphocytes activated by antigenic stimuli and searched for it. The present inventors have already found that culture supernatant of peripheral T lymphocytes from allergic patients with eosinophilia shows eosinophil chemotactic activity, and successfully established a T cell line, STO-2, which constantly produces culture supernatant exhibiting eosinophil chemotactic activity (Lymphokine Cytokine Res. 10, 481-486, 1991; Lymphokine Cytokine Res. 11, 331-338, 1992). The present inventors extracted messenger RNA from the T cell line STO-2 and conducted reverse transcription to prepare a cDNA expression library. Meanwhile, the eosinophil chemotactic factor was partially purified from STO-2 culture supernatant, and was used to immunize rabbits to obtain an antibody absorbing this eosinophil chemotactic activity. Using this antibody, the above cDNA expression library was screened to obtain 32 clones reactive with the antibody. These 32 clones were transfected into COS cells to investigate the eosinophil chemotactic activity in the culture supernatant, and three independent clones were found to be positive. Complete sequences of the CDNA corresponding to these three clones were determined by Sanger's method, and all these clones were found to encode an identical protein consisting of 323 amino acids.

This eosinophil-specific chemotactic factor has a molecular weight of 36 kDa, calculated on the basis of the nucleotide sequence of the cDNA, and the isoelectric point of 8.1. Homology search using computers and databases showed this cDNA was similar to the sequence of galectin-9, a member of sugar binding proteins. In general, galectins are galactose (β galactoside)- binding lectin with thiol requirement (European Journal of Biochemistry 243, 543-576, 1997). The present inventors named this eosinophil-specific chemotactic factor similar to galectin-9, "ecalectin".

As used herein, the term "ecalectin" refers to a protein defined as (a) or (b) below:
(a) A protein comprising the amino acid sequence of SEQ ID NO: 2.
(b) A protein comprising the amino acid sequence of SEQ ID NO: 2 in which one or more amino acids are deleted, replaced or added and retaining activity of increasing eosinophil chemotaxis.

Namely, ecalectin includes a protein functionally equivalent to the ecalectin protein that comprises the amino acid sequence of SEQ ID NO: 2 and has activity of increasing eosinophil chemotaxis. In other words, ecalectin includes a protein that comprise the amino acid sequence of the ecalectin protein of SEQ ID NO: 2 in which one or more amino acids are deleted, replaced or added and has the activity of increasing eosinophil chemotaxis. The number of amino acids to be mutated is not limited as long as the function of the ecalectin protein is retained, and the number is generally 50 amino acids or less, preferably 20 amino acids or less, more preferably 10 amino acids or less, and most preferably 3 amino acids or less. Any amino acid can be substituted as long as the ecalectin protein retains its function.

As used herein, the term "DNA encoding ecalectin" refers to any one of the DNA defined as (a) through (e):
(a) DNA comprising the nucleotide sequence of SEQ ID NO: 1;
(b) DNA encoding a protein that hybridizes to DNA comprising the nucleotide sequence of SEQ ID NO: 1 and has activity of increasing eosinophil chemotaxis;
(c) DNA encoding a protein that has high homology to the DNA comprising the nucleotide sequence of SEQ ID NO: 1 and has activity of increasing eosinophil chemotaxis;
(d) DNA encoding the amino acid sequence of SEQ ID NO: 2; and
(e) DNA encoding a protein comprising the amino acid sequence of SEQ ID NO: 2 in which one or more amino acids are deleted, replaced or added and has activity of increasing eosinophil chemotaxis.

As methods for preparing DNA encoding a functionally equivalent protein, various mutagenesis methods are well known to a person skilled in the art. More specifically, a person skilled in the art can use, for example, site-directed mutagenesis system by PCR (Takara, Kyoto) and by oligonudeotide (Takara) to design genes in which amino acids in the ecalectin protein comprising the amino acid sequence of SEQ ID NO: 2 is appropriately replaced, thereby generating DNA that encodes a protein functionally equivalent to the ecalectin protein. DNA mutation resulting in amino acid mutation in the protein that is encoded by the DNA is a common event in nature, thus, the present invention includes DNA encoding a spontaneous mutant functionally equivalent to ecalectin.

As a method for isolating DNA encoding functionally equivalent proteins, hybridization techniques are frequently used. More specifically, a person skilled in the art generally uses a hybridization technique to isolate highly homologous DNA by using a DNA sequence encoding the ecalectin protein of SEQ ID NO: 1 or its part as a probe. DNA of the present invention also includes such DNA that hybridizes to the DNA sequence of SEQ ID NO: 1, which encodes the ecalectin protein, and also encodes a protein with the activity of increasing eosinophil chemotaxis. Such DNA includes DNA derived from vertebrates such as non-human mammals, including, mouse, rat, Guinea pig, pig, cattle, monkey, fish, and reptile; invertebrates, for example, insects and vermins; microorganisms, for example, fungi, *E*. *coli*, and archaebacterium such as halobacterium; plants, such as tobacco and *Arabidopsis* *thaliana*; and viruses, for example, bacteriophage, adenovirus, and retrovirus. The hybridization conditions for isolating DNA encoding a protein functionally equivalent to ecalectins are generally about "60°C, 2 x SSC, 0.1% SDS", preferably about "65°C, 2 x SSC, 0.1% SDS", more preferably "65°C, 0.2 x SSC, 0.1% SDS", and the most preferably about "68°C, 0.2 x SSC, 0.1% SDS". DNA with higher homology can be obtained, as the hybridization conditions become more stringent. As used herein, the term "stringent hybridization conditions" refers to the conditions more stringent than those of "65°C, 2 x SSC, 0.1% SDS". DNA used in the present invention also includes DNA that has high homology to the nucleotide sequence of SEQ ID NO: 1 and encodes a protein with activity of increasing eosinophil chemotaxis. High homology refers to, generally 50% or higher, preferably 70% or higher, more preferably 85% or higher, and most preferably 95% or higher homology with the DNA of SEQ ID NO: 1. Homology search can be performed using a program, for example, DNASIS (Hitachi Soft Engineering).

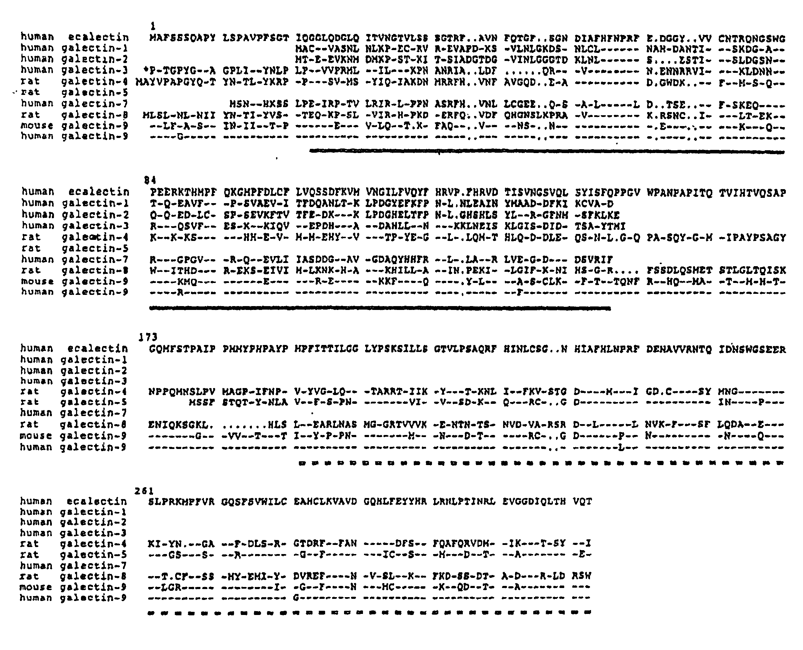

A cDNA clone obtained enables ecalectin production by using cultured cells or insect cells into which the cDNA clone has been introduced, as well as from T lymphocytes activated by antigenic stimuli. The comparison between the primary amino acid sequences of ecalectin and those of known galectins is shown in Figure 1.

Ecalectins can be purified utilizing their characteristics as lectin, with, for example, a lactose column.

The present inventors also revealed that chemotactic activity of ecalectin on neutrophils, lymphocytes and monocytes is low or absent. On the other hand, the chemotactic activity of ecalectin on eosinophils is about 4 to 5 times as high as that of IL-5 known as a compound comprising a chemotactic activity specific to eosinophils.

Investigation for organs in which ecalectin is found has revealed that ecalectin transcripts are found in those organs in which lymphocytes and eosinophils concomitantly exist, such as lung, stomach, and small intestine, and that among lymphatic tissues, ecalectin is strongly expressed in spleen, lymph node, thymus, bone marrow, fetal liver, etc.

It has also been revealed that ecalectin is expressed in peripheral leukocytes, and that the ecalectin production in T lymphocytes from antigen-sensitized individuals is remarkably increased when the T lymphocytes are stimulated by the antigen.

As a result of the above investigation, ecalectin, a member of the galectin family, has been revealed to be a factor having the eosinophil chemotactic activity extremely specific for eosinophils. Therefore, a galectin can be used as an effective ingredient in an agent to increase eosinophil chemotaxis. The finding of the present invention that galectins increase eosinophil chemotaxis suggents that the chemotaxis of eosinophils can be reduced by an agent comprising as an effective ingredient a compound that inhibits the galectin activity to increase the eosinophil chemotaxis. The present inventors also provide a method for screening compounds that are candidates as an effective ingredient of this agent. Specifically, a compound that decreases the eosinophil chemotaxis can be screened by selecting a test compound that reduces the eosinophil chemotaxis induced in the presence of a galectin and eosinophils, compared with that induced in the absence of the test compound.

More specifically, the present invention includes:
(1) An agent for increasing eosinophil chemotaxis, said agent comprising a galectin as an effective ingredient;
(2) An agent for decreasing eosinophil chemotaxis, said agent comprising, as an effective ingredient, a compound that inhibits the galectin activity to increase eosinophil chemotaxis;
(3) The agent of (1) or (2), wherein said galectin is ecalectin;
(4) A method for determining whether a test compound reduces eosinophil chemotaxis or not, said method comprising monitoring eosinophil chemotaxis induced in the presence of said test compound together with a galectin and eosinophils, compared with that induced in the absence of said test compound;
(5) A method for screening for a compound that reduces eosinophil chemotaxis, said method comprising the steps of:
   (a) monitoring eosinophil chemotaxis in the presence of a test compound together with a galectin and eosinophils, compared with that induced in the absence of said test compound, and
   (b) selecting a compound that reduces the eosinophil chemotaxis obtained in the step (a);
(6) The method of (4) or (5), wherein said galectin is ecalectin;
(7) A kit comprising a galectin, wherein said kit is provided for the screening of (5); and
(8) The kit of (7), wherein said kit further comprises eosinophils.

As obvious from Figure 1, the homologous regions in the galectin at the amino acid level show 40 to 90% or higher homology with regard to at least the deduced carbohydrate binding sites (the regions shown with bold lines and bold dot lines indicate the primary and secondary carbohydrate binding sites, respectively)., This fact implies that binding of galactose present on the eosinophil surface to these carbohydrate binding sites may relate to eosinophil chemotaxis. Therefore, as a galectin used for this invention, any known galectins as well as compounds newly classified as galectin can be used as long as they has eosinophil chemotactic activity. Examples of galectins include human galectin-1, human galectin-2, human galectin-3, rat galectin-4, rat galectin-5, human galectin-6, rat galectin-8, mouse galectin-9, human galectin-9, etc. Preferably, galectins are those classified as galectin-9, and more preferably galectin is ecalectin. The above galectins are preferably modified by glycosylation, more preferably by mammalian-type glycosylation, and most preferably by human-type glycosylation.

As a source from which galectins are obtained and purified, peripheral blood sensitized T lymphocytes stimulated by antigens can be used. Cells constantly producing galectins, for example, STO-2 cell line, can also be used as the source. Alternatively, recombinant galectins can be prepared by introducing a gene encoding a galectin into host cells, such as animal cells and microorganism, via appropriate vectors and expressing it. Recombinant galectins can be obtained by collecting the expression products from the galectin producing cells. Host cells are preferably cells in which expression products undergo glycosylation, more preferably cells derived from mammals, and most preferably human cells.

Any DNA can be used as DNA encoding galectin, as long as it comprises a structural gene of a galectin, including cDNA, genomic DNA, viral DNA, and chemosynthetic DNA. cDNA can be prepared, for example, by designing primers in reference to a DNA sequence of a galectin used for this invention (e.g. SEQ ID NO: 2) by chemical synthesis and conducting PCR. To prepare DNA encoding a galectin from genomic DNA, for example, "Qiagen genomic DNA kits" (Qiagen, Germany, Hilden), and the like can be used. The nucleotide sequence of the DNA obtained can be determined by a method, such as Sanger's. Galectin DNA would be applied to gene therapy, as well as preparation of recombinant proteins. For example, gene therapy targeting a galectin producing gene to inhibit galectin production, would be effective for treating various allergic disorders, which are typical delayed inflammations caused by eosinophils, including numerous types of bronchial asthma, allergic rhinitis, and atopic dermatitis.

Galectin expression vectors used in the present invention include various vectors such as those for expressing a galectin *in vivo*, and those for preparing recombinant proteins, according to the purposes. Examples of the vectora used for expressing a galectin *in vivo* (for example, for gene therapy), include adenovirus vectors, retrovirus vectors, etc. When constructing an expression vector, it is important to optionally set control elements, which efficiently transcribe/translate a galectin structural gene, for example, by inserting a transcription promoter such as CMV promoter and SV40 promoter upstream the galectin gene, by inserting Kozak consensus sequence at the translation initiation site, or by inserting transcription terminators or poly (A) signals downstream the galectin structural gene. It is also important to alter the codon usage of a galectin structural gene in an appropriate form for hosts.

For a vector for preparing a recombinant protein, transcription/translation control elements appropriate for hosts are preferably set at the upstream or downstream region of the galectin structural gene. Similar to the above, appropriate modification of the codon usage of the galectin structural gene is important. For example, when *E*. *coli* is used as a host, codons in the galectin structural gene are preferably modified, referring to the frequency of codon usage in *E*. *coli*. Hosts preferably used include *E*. *coli*, yeast, and insect cells. When mammalian cell lines, such as CHO, COS, and 293 cells, are used, an expression vector can be introduced into the cells using, for example, Lipofectin reagent.

Transformants effectively usable in the present invention include, for example, those harboring a vector as described above which carries galectin DNA epichromosomally, and those in which the DNA of the present invention is integrated into the host genome. Their form of existence is not limited as long as the galectin DNA is retained in the cells in an expressible manner (including the inducible expression). Any cell can be used as a cell into which a vector carrying galectin DNA is introduced. For example, for gene therapy, various cells can be used as a target cell according to disorders. For preparing a galectin, for example, *E*. *coli*, yeast, animal cells, and insect cells can be used. A vector can be introduced into a cell by, for example, calcium phosphate method, electroporation, heat shock, or an introduction method using cationic lipid, such as Lipofectin etc. Transformants thus prepared can be collected, and galectin proteins recombinantly expressed can be isolated and purified by a standard method. In addition, as being a member of lectins, a galectin can be effectively purified using, for example, a lactose column (Seikagaku Corporation, Tokyo).

An agent of the present invention for increasing eosinophil chemotaxis, which comprises a galectin as an effective ingredient, can be directly used as a reagent, or as a therapeutic or preventive drug. The agent of this invention can be prepared into any form, including liniment or spray including percutaneous or pernasal sorbent, tablet, granule, powder and common aqueous injections as long as the agent retains its activity to increase eosinophil chemotaxis. Any forms of the agent can be mixed with buffers, stabilizers, preservatives, antioxidants, painkillers, solubilizers, or appropriate carriers or media generally used in the known pharmaceutical production methods as long as the agent retains its activity to increase eosinophil chemotaxis. For preparing pharmaceuticals suitable for administration *in vivo*, including injections, percutaneous sorbents, pernasal sorbents, and oral agents, preferably injections, carriers or media such as sterilized water, isotonic solution such as glucose, saline, can be optionally combined with, for example, vegetable oil (for example, sesame oil, olive oil), coloring agents, emulsifiers (for example cholesterol), suspension (for example, gum arabic), detergents (for example, polyoxyethylene hydrogenated castor oil detergents), solubilizers (for example, sodium phosphate), stabilizers (for example, sugar, sugar alcohol, albumin), or preservatives (for example paraben). Injectable preparations can be provided, for example, in the form of, lyophilizate or aqueous injection, or in a form trapped in an osmotic pump. The agent of the present invention thus prepared can be administered to mammals such as humans. Daily osage varies depending on the conditions of subjects. In intravenous administration to an adult, dosage range generally from about 0.001 to about 100 mg, preferably from about 0.01 to about 10 mg, and more preferably from about 0.1 to 1 mg. The agent of the present invention can be used, for example, to transfer an appropriate amount of eosinophils in a tissue to a patient or a diseased part with a very small amount of eosinophils in the tissue. For example, the agent of the present invention can be applied to a diseased part of, for example, cancer and malignant tumor to diminish the lesion

For administering the agent into a diseased part, such as cancer and malignant tumor, the lesion can be exposed using a surgical method so as to apply the agent of the present invention directly to the diseased part. When a damaged site is close to blood vessels, the agent of the present invention can be applied through a catheter.

Cancerous lesions or malignant tumors can also be diminished, using a vector for gene therapy that carries a galectin gene. Specifically, common viral vectors, such as retrovirus and adenovirus vectors, or non-viral vectors, such as liposome, that carry a transcription promoter and a galectin gene, can be used as vectors for gene therapy. These vectors for gene therapy can be directly administered to a diseased part. Alternatively, cultured cells into which these vectors are introduced can be transplanted to the diseased part. Those cultured cells should give good expression of galectin, preferably do not show immunogenicity against the host. Most preferably, the cells present at or near the lesion can be used.

The agent of the present invention can be used as a regent for screening for compounds inhibiting the galectin activity to increase eosinophil chemotaxis.

The agent for reducing eosinophil chemotaxis comprising as an effective ingredient a compound that inhibits the galectin activity to increase eosinophil chemotaxis, can be directly used as a reagent, or as a therapeutic or preventive drug. The agent of this invention can be prepared into any form including liniments, or spray including percutaneous or pernasal sorbents, tablet, granule, powder or common aqueous injections, as long as the agent retains its activity to reduce eosinophil chemotaxis. Any forms of the agent can be mixed with buffers, stabilizers, preservatives, antioxidants, pain-killers, solubilizers, or appropriate carriers or media generally used in the known pharmaceutical production methods as long as the agent retains its activity to reduce eosinophil chemotaxis. For preparing pharmaceuticals suitable for administration in vivo, including injections, percutaneous sorbents, pernasal sorbents, and oral agents, preferably injections, carriers or media such as sterilized water, isotonic solution such as glucose, saline, can be optionally combined with, for example, vegetable oil (for example, sesame oil, olive oil), coloring agents, emulsifiers (for example cholesterol), suspension (for example, gum arabic), detergents (for example, polyoxyethylene hydrogenated castor oil detergents), solubilizers (for example, sodium phosphate), stabilizers (for example, sugar, sugar alcohol, albumin), or preservatives (for example paraben). Injectable preparations can be provided, for example, in the form of, lyophilizate or aqueous injection, or in a form trapped in an osmotic pump. The agent of the present invention thus prepared can be administered to mammals such as humans. Dosage varies depending on the conditions of subjects. In intravenous administration to an adult, daily dosage range generally from about 0.001 to about 100 mg, preferably from about 0.01 to about 10 mg, and more preferably from about 0.1 to 1 mg. The agent of the present invention can be used for treatment or prevention of allergic diseases attributed to eosinophils. Examples of allergic diseases attributed to eosinophils include various kinds of bronchial asthma, allergic rhinitis, and atopic dermatitis.

To screen for compounds that inhibit the galectin activity of increasing eosinophil chemotaxis, for example, the methods described in the references (Lymphokine Cytokine Res. 10, 481-486, 1991; Lymphokine Cytokine Res. 11, 331-338, 1992) can be applied. For example, eosinophils of CD 16- are purified from the peripheral blood from normal volunteers by discontinuous density-gradient method using a Percoll (Pharmacia, Piscataway, New Jersey), followed by magnetic antibody treatment using anti-CD16 immunoglobulin (DAKO. A. S. Glostrup, Denmark). In a bilayer plate intervened by a polyvinylpyrrolidone-free membrane (a standard pore size of 5 micron), 0.5 to 1 x 10⁶ cells/ml of human eosinophils are placed on the upper layer and various concentrations of the test compound and ecalectin at an optimal concentration for eosinophil chemotaxis are placed on the lower layer. The plates are then incubated at 37°C for 1 to 2 hours under 5% CO₂₋Subsequently, the membrane separating the two plates is removed and placed in DiffQuick stain (Baxter Health Care, McGaw Park, Illinois) to stain eosinophils, and eosinophils which were passing through the membrane are counted under a microscope. Thus, compounds inhibiting the activity to increase eosinophil chemotaxis can be screened by selecting the compounds that decrease eosinophil chemotaxis.

### Brief Description of the Drawings

Figure 1 shows the comparison between structures of ecalectin cDNA and other galectins. Amino acids in the figure are indicated by a one-letter code described on page 1468 of "Dictionary of Biochemistry, Second Edition (Tokyo Kagakudojin, 1990)".
Figure 2A is electorophoretic patterns showing the expression level of ecalectin transcripts in various tissues; 1: brain, 2: heart, 3: kidney, 4: spleen, 5: liver, 6: peripheral lymphocytes, 7: lung, 8: small intestine, 9: muscle, 10: stomach, 11: testicle, and 12: placenta.
Figure 2B is electorophoretic patterns showing a control experiment for measuring the relative amount of RNA. G3PDH was used as a probe.
Figure 3A is electorophoretic patterns indicating the expression level of ecalectin transcripts in lymphoid tissues; 1: spleen, 2: lymph node, 3: thymus, 4: peripheral leukocytes, 5: bone marrow, and 6: fetal liver.
Figure 3B is electorophoretic patterns, showing a control experiment for measuring the relative amount of RNA. G3PDH was used as a probe.
Figure 4A is electorophoretic patterns showing the increased production of ecalectin transcripts by antigenic stimuli.
Figure 4B is electorophoretic patterns showing a control experiment for measuring the relative amount of RNA. G3PDH was used as a probe.
Figure 5 shows that ecalectin has concentration-dependent eosinophil chemotactic activity. The horizontal axis indicates the concentration of ecalectin, and the vertical axis the number of stained eosinophils that were passing through the pores of the membrane. Black and white circles indicate the results of ecalectin and negative control using PBS, respectively.
Figure 6 shows that ecalectin does not cause neutrophil chemotaxis. The vertical axis indicates the number of the stained neutrophils that were passing through the pores of the membrane.
Figure 7 shows that ecalectin does not cause lymphocyte chemotaxis. The vertical axis indicates the number of the stained lymphocytes which were passing through the pores of the membrane.
Figure 8 shows that the recombinant ecalectin does not cause monocyte chemotaxis. The vertical axis indicates the number of the stained monocytes which were passing through the pores of the membrane.

### Best Mode for Carrying out the Invention

The present invention is illustrated in detail below with reference to examples, but should not be construed as being limited to the examples.

### Example 1: Partial purification of ecalectin and preparation of polyclonal anti-serum comprising ecalectin specific antibody

A cell line STO-2, which was obtained by transformation of T cells derived from a patient with eosinophila with human T cell leukemia virus (Lymphokine Cytokine Res. 10, 481-486, 1991) was used as a source material. FACS analysis confirmed this cell line was derived from T cell lines. Specifically, STO-2 expressed CD2, CD3, CD4, CD5, and CD8, but did not express CD16, CD19 or Leu7, markers for granulocyte/macrophage and B cells.

Culture supernatant of STO-2 cells cultured in SF-02 serum-free medium (Sanko Pure Chemicals, Tokyo) supplemented with 0.1% human serum albumin, 100 U/ml of IL-2 (Tokushima Research Institute, Tokushima), 50 µM 2-mercaptoethanol, 100 µg/ml streptomycin, 100 U/ml penicillin, and 5 µg/ml Fungizone for about 72 hours, was adjusted to pH 5 using 50 mM sodium acetate buffer. Five litters of the condition medium thus obtained were passed through several CM Sepharose columns (Pharmacia) (2.6 x 40 cm) equilibrated with 50 mM sodium acetate (pH 5.0). By washing these ionic exchange columns with equilibration buffer, albumin, a main contaminating protein contained in the condition medium, was removed. An eosinophil chemotactic factor adsorbed in the column was eluted using 20 mM sodium phosphate buffer (pH 7.0) containing 0.1 M sodium chloride. The eosinophil chemotactic factor was isolated and purified using as an index an eosinophil chemotactic activity in each fraction, which was measured by the method described in Lymphokine Cytokine Res. 10, 481-486, 1991 and Lymphokine Cytokine Res. 11, 331-338, 1992. About 300 ml of the eluate was dialyzed against 1% glycine solution and subjected to the isoelectric point focusing for isolation. The fraction of 40 ml comprising pI 7 to 8 proteins was collected on Rotofor system (Bio-Rad, Hercules, California). This fraction in which the eosinophil chemotactic factor was concentrated was concentrated to about 1/10 using an ultrafiltration device and YM-5 membrane (Amicon, Lexington, Massachusetts). This was dialyzed against PBS and fractionated by 1 x 30 cm Superlose-12 column (Pharmacia) equilibrated with PBS. The calibration of this gel filtration column indicated that most ECA activities existed in 30 to 40 kDa fractions. The fraction in which the eosinophil chemotactic factor was concentrated was precipitated with 60% saturated ammonium sulfate. This precipitate was dissolved in 20 mM sodium phosphate buffer (pH 7.0) containing 30% ammonium sulfate to fractionate with 4.6 mm x 7.5 cm reversed phase column TSK gel Phenyl-5PW (Tosoh, Tokyo) equilibrated with the same buffer. Ammonium sulfate in the buffer was decreased to 30 to 0% by the linear gradient treatment. The finally obtained active fraction was used to sensitize rabbits with Freund's complete adjuvant. After administrations of Freund's incomplete adjuvant several times, serum was collected and used as a polyclonal anti-serum comprising an "anti-eosinophil chemotactic factor-specific antibody".

### Example 2: Screening of a cDNA library prepared from T cell line STO-2 and positive clones

A STO-2 cell cDNA expression library was prepared using ZAP Express™ cDNA Gigapack cloning kit (Stratagene, La Jolla, California). Specifically, about 5 µg of poly (A)⁺ mRNA was collected using Fast Track RNA Isolation kit (Invitrogen, San Diego, California) from 1 x 10⁸ STO-2 cells. A first DNA strand was synthesized using Moloney mouse leukemia virus-derived reverse transcriptase and 50-mer oligo dT primer 
(5'-
GAGAGAGAGAGAGAGAGAGAACTAGTCTCGAGTTTTTTTTTTTTTTTTTT-3';
containing a restriction enzyme XhoI site: SEQ ID NO: 3).
 This DNA strand production reaction was conducted in the presence of 5-methyl CIP so as to methylate a XhoI site in cDNA. An EcoRI-adapter was ligated to 5'-end of this cDNA. This was cleaved with a restriction enzyme XhoI (Stratagene), and size-fractionated using 1 ml of Sephacryl S-500 HR. (Gibco) gel filtration column equilibrated with 0.1 mM EDTA, 25 mM NaCl, and 10 mM Tris-HCl (pH 7.5). Fractions of 500 bp (base pairs) or more were pooled, and cloned to a bacteriophage expression vector of a kit (ZAP Express™) one after another. This was incorporated into a phage particle using Gigapack III Gold extract. The phage particles obtained were transfected into *E*. *coli* XL1-Blue MRF' to obtain a cDNA library with about 1 x 10¹¹ pfu/ml (plaque forming units per 1 ml) titer. Immunoreaction positive clones in this STO-2 cell cDNA library were screened using picoBlue^{TM} immunoscreening kit (Stratagene) and anti-serum against human ecalectin obtained in Example 1 (about 10 ng/ml) as a probe.

As a result, 32 immunoreaction positive clones were obtained. A construct in which cDNA was inserted into vector pBK-CMV capable of expressing the cDNA in mammalian cells was cleaved out from these clones using ExAssist/XLOLR system (Stratagene). The plasmid DNA in these positive clones was purified using Plasmid Miniprep Isolation kit (Qiagen, Santa Clara, California). About 5 µg of this purified plasmid was transfected into COS cells using the DEAE-dextran method (Mol. Cell. Biol. 4, 1641-1643, 1984). As a plasmid for a negative control, pCMV β-gal cDNA (Stratagene) was used.

Transfectants were cultured in RPMI-1640 medium with 10% fetal calf serum and 50 µM 2-mercaptoethanol at 37°C for 3 days, and the culture supernatant was subjected to ECA (eosinophil chemotaxis) test.

The eosinophil chemotaxis was measured by following the method described in the reference (Lymphokine Cytokine Res. 10, 481-486, 1991; Lymphokine Cytokine Res. 11, 331-338, 1992). Specifically, eosinophils of CD16- were purified from the peripheral blood of normal volunteers by discontinuous density-gradient method using Percoll (Pharmacia, Picataway, New Jersey), and the following anti-CD16 immunoglobulin (DAKO. A. S, Glostrup, Denmark) magnetic antibody treatment. Purity of the obtained eosinophils was 97% or higher, and living cells were 95%. Chemotaxis was measured using a bilayered 48-well plate (NeuroProbe, Cabin John, Maryland) mediated through a polyvinylpyrrolidone-free membrane (a standard pore size of 5 micron). Chemotactic factors in various concentration, and 0.5 x 10⁶ to 1 x 10⁶ cells/ml human eosinophils were placed on the lower and upper layers, respectively, and incubated under 5% carbon dioxide at 37°C for 1 to 2 hours. Then the membrane separating two plates was removed, and eosinophils were stained by placing them in DiffQuick stain (Baxter Health Care, McGaw Park, Illinois) to count the eosinophils which were passing through the membrane under a microscope. All assays were conducted three times.

As a positive control, human C5a (Sigma, Saint Louis, Missouri), IL-5 (Genzyme, Cambridge, Massachusetts), RANTES (Genzyme), and eotaxin-1 (Seikagaku-Kogyo, Tokyo) were used.

Culture supernatant of transfectants was analyzed, and three clones showed sufficient ECA activity in the eosinophil chemotaxis test *in vitro*.

### Example 3: Determination and analysis of nucleotide sequences

The DNAs of three independent clones obtained in Example 2 were sequenced by Sanger method (Proc. Natl. Acad. Sci. USA 74, 5463-5467, 1997) to find to encode an identical protein. A DNA consensus sequence and its deduced amino acid sequence are shown in SEQ ID NO: 1 and SEQ ID NO: 2, respectively. A deduced open reading frame was thought to correspond to nucleotide Nos. 60 to 1028 in SEQ ID NO: 1, and expected to encode a 36 kDa protein encoding 323 amino acids. The present inventors named this cloned eosinophil chemotactic factor ecalectin. Nucleotide Nos. 1576 to 1581 in SEQ ID NO: 1 are a typical polyadenylation signal. In this clone, 59 nucleotides at 5' untranslated region, and 560 or more nucleotides at 3' untranslated region exist. In a transcript with short half life, a repetitive sequence was often observed in 3' untranslated region (Cell, 46, 659-667, 1986). "AUUUA" motif found in transcripts of eotaxin-1 and many chemokines and cytokines, does not exist in 3' untranslated region of the ecalectin cDNA. Instead, four repetitive "CCCUCC" motifs are found at nucleotide Nos. 1341 to 1484 in 3' untranslated region. A natural ecalectin is a secretory protein. Amino acid sequences deduced from the STO-2-derived transcripts comprise many Phe, Leu, and Val residues, however, a hydrophobic signal peptide at the amino terminus found in many secretory proteins was not found. pI of ecalectin was estimated as 8.1. In the ecalectin, four sites to which Asn binding type sugar chains can be added exist at amino acid Nos. 34, 79, and 137 of SEQ ID NO: 2.

### Example 4: Analysis of homologous regions between ecalectin and galectins

Using the homology search of GenBank, homologous regions of proteins and nucleic acids were analyzed. It was found that ecalectin does not comprise a homology with known cytokines or chemokines, but comprises a homology with galectins (J. Biol. Chem. 269, 20807-20810, 1994). Especially, ecalectin comprises a very high homology with human- and mouse-derived galectin-9 (J. Biol. Chem. 272, 6416-6422, 1997; J. Biol. Chem. 272, 6078-6086, 1997). Figure 1 shows the result of analyzing homologous regions between already reported galectins and ecalectin. This result suggests that ecalectin is a protein classified into galectin-9 of galectins.

### Example 5: Distribution of ecalectin in organs

Ecalectin-existing organs were investigated. From various human organs, mRNA was obtained, and organs in which its mRNA was expressed were examined by the blot analysis.

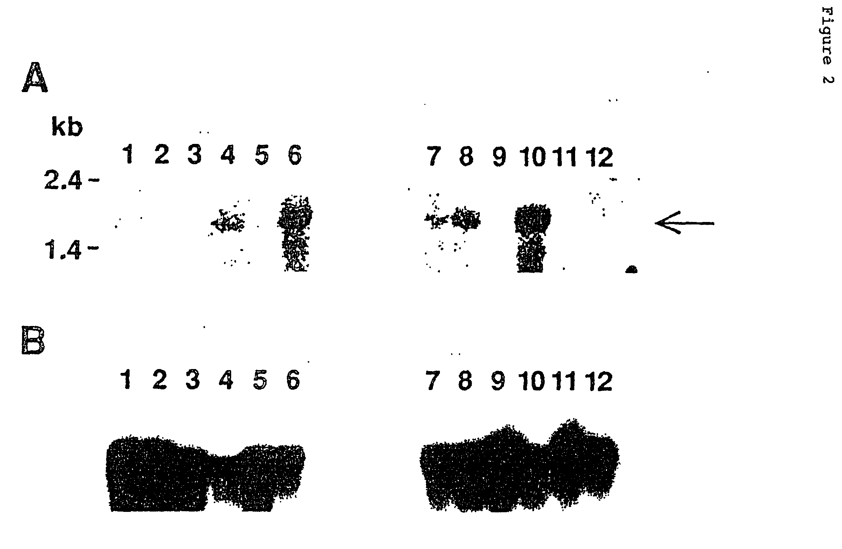

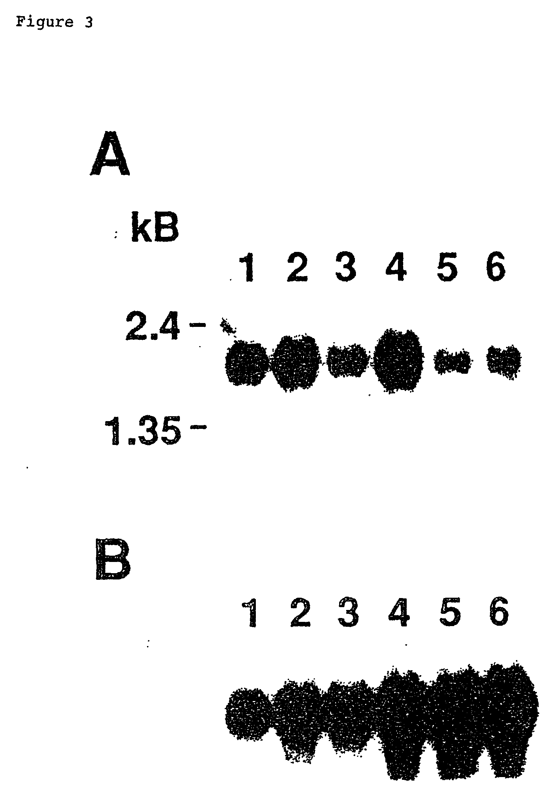

A blot containing total RNA (Origene Technologies, Rockville, Maryland) or a blot with poly (A)+ RNA (Clontech, Palo Alto, California) derived from various human organs was analyzed using a radiolabeled ecalectin cDNA probe. The RNA blots reacted with the probe at 68°C for 2 hours were washed with 2 x SSC containing 0.1% SDS at room temperature for 15 min twice, and further washed with 0.1 x SSC containing 0.1% SDS at 60°C for 30 min twice. Autoradiographic images were analyzed using the BAS-200032P imaging device (Fuji Film, Tokyo). Figures 2 and 3 show the result. The transcripts were observed in the organs in which lymphocytes and eosinophils are considered to exist together, such as lung, stomach, and small intestine (Figure 2A; expression of ecalectin in 1. brain, 2. heart, 3. kidney, 4. spleen, 5. liver, 6. peripheral lymphocyte, 7. lung, 8. small intestine, 9. muscle, 10. stomach, 11. testicle, and 12. placenta), and among lymphoid tissues, the strong expression was found in spleen, lymph node, thymus, bone marrow, fetal liver, etc. (Figure 3A shows the ecalectin expression in; 1. spleen, 2. lymph node, 3. thymus, 4. peripheral leukocyte, 5. bone marrow, and 6. fetal liver.) As the RNA used in the experiment of Figure 3 was poly (A)+ RNA, the RNA amount was nearly 1000 times as much as the result of Figure 2 using the total RNA. Figures 2B and 3B are the control experiments for confirming the relative amount of RNA loaded in each lane. The blots (membranes) used in these control experiments were reacted with a human glyceraldehyde 3-phosphate dehydrogenase (G3PDH) cDNA probe of 548 bp.

### Example 6: Induction of ecalectin by antigenic stimulus to T cells in the peripheral blood of a patient sensitized with mite antigen

Since ecalectin was expressed in tissues containing T lymphocytes, such as the peripheral blood, the experiment was conducted to confirm whether the production would be increased or not when T lymphocytes were stimulated by an antigen.

Mononuclear cells were isolated from the peripheral blood from volunteers sensitive to the mite Dermatophagoides farinae (Df) antigen by the Ficoll-Hypaque density-gradient centrifugation. Cells (5 x 10⁶ cells/ml) were cultured for 48 hours in the RPMI-1640 medium containing 5% fetal calf serum or RPMI-1640 medium with 5% fetal calf serum and 5 µg/ml Df.

Total RNA was extracted from these cells using RNeasy Midi kits (Qiagen). About 5 µg of total RNA was denatured and electrophoresed on a 1.4% agarose/formamide gel. RNA separated in gel was blotted onto the Hybond^{TM} nylon membrane (Amersham, Arlington Heights, Illinois) (Proc. Natl. Acad. Sci. USA 77, 5201-5205, 1980). After the crosslinking-treatment with UV, the membranes were treated with QuickHyb (Stratagene) containing 40 µg/ml denatured salmon sperm DNA (Stratagene) at 68°C for 30 min, and reacted in the same buffer with the radio-labeled ecalectin probe for 2 hours. To confirm the relative amount of RNA loaded in each lane, the used blots (membranes) were reacted with the 548 bp human glyceraldehyde 3-phosphate dehydrogenase (G3PDH) cDNA.

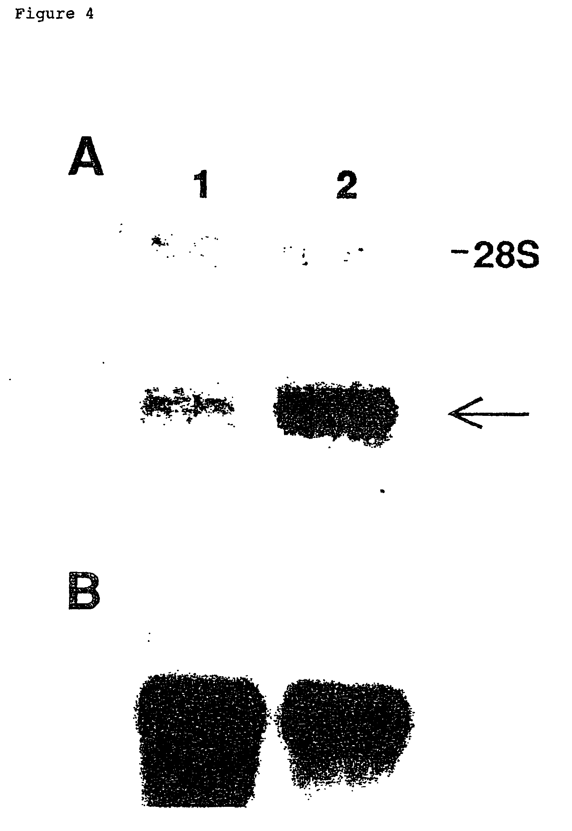

Figures 4A and 4B show the results. The peripheral monocytes derived from allergic patients produced a small amount of ecalectin mRNA. These cells stimulated with the Df antigen for 48 hours, however, produced 50 to 90 times as much mRNA as the original cells (Figure 4A). It has been also found that the cells which received such antigenic stimulus secreted ecalectin in the culture medium. These findings strongly suggest that T lymphocytes of allergic patients against a certain specific antigen, produce a large amount of ecalectin by the antigenic stimulus. In other words, ecalectin would have an extremely important role in increasing eosinophils in a tissue at the site of allergy where excessive immunoreaction against a specific antigen occurs.

Figure 4B shows a control experiment measuring the relative amount of RNA loaded in each lane, using the G3PDH cDNA described in Example 6 as a probe.

### Example 7: Preparation and purification of recombinant ecalectin using insect cells

The ecalectin cDNA which was inserted into pBK-CMV obtained in Example 2 was cut out, isolated, and ligated downstream of a polyhedrin gene promoter of pVL1393 (PharMingen, San Diego, California) capable of expressing a gene in *Spondoptera frugipenda* 9 (Sf9) insect cells. This plasmid was purified by cesium chloride-ethidium bromide equilibrium density-gradient ultracentrifugation, and incorporated into Sf9 cells by calcium phosphate method, with Baculo Gold™ DNA (PharMingen).

By the plaque assay method, the baculovirus containing ecalectin cDNA was identified, isolated, amplified, and then infected to Sf-9 cells again. The infected cells were cultured in TNM-FH medium containing 10% heat-treated FCS at 27°C for 3 days, and about 7 x 10⁶ cells thus obtained were ultrasonically disintegrated in the presence of a protease inhibitor. A soluble fraction was subjected to 1 ml of a lactose column (Seikagaku Corporation, Tokyo), and eluted with TBS buffer containing 200 mM lactose to collect the recombinant ecalectin purified in a single band. In insect cells, most ecalectin exist within a cell.

### Example 8: Specific reactivity of ecalectin against eosinophils

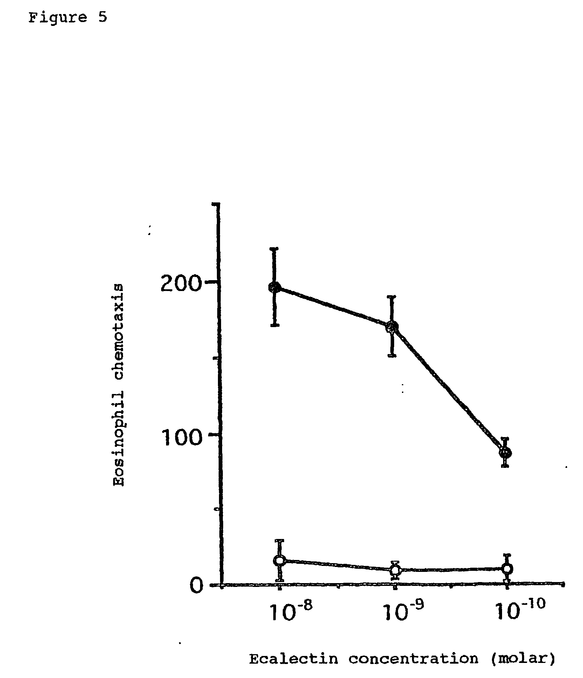

Using the test procedure described in Example 2 for measuring chemotactic activity, whether the recombinant ecalectin purified in Example 7 comprises eosinophil-specific chemotactic activity or not was examined. Figures 5, 6, 7, and 8 show the results.

Ecalectin was found to comprise the eosinophil chemotactic activity like a positive control eotaxin-1. As obvious in Figure 5, ecalectin showed the strong eosinophil chemotactic activity concentration-dependently. The maximum activity was about four times as strong as that of IL-5.

To confirm whether the chemotactic activity of ecalectin is specific to eosinophils or not, the following experiment was conducted.

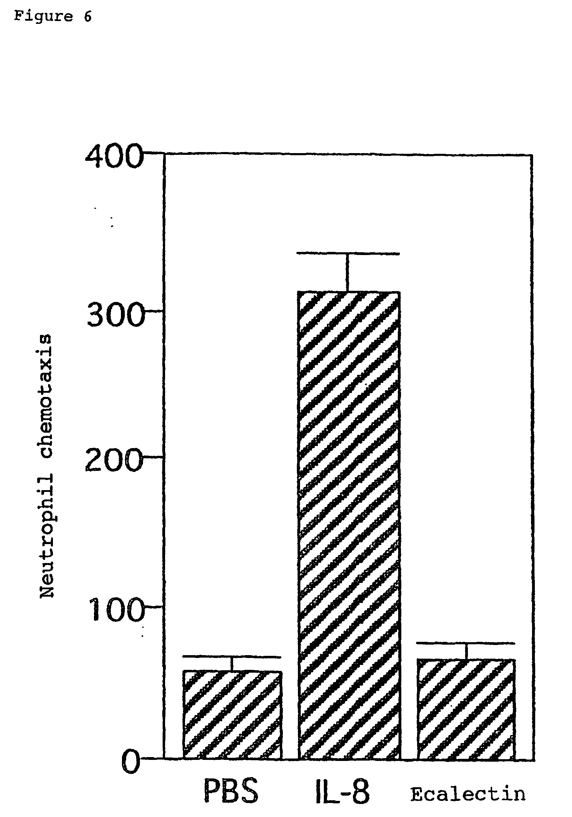

In the chemotaxis experiment for eosinophils described in Example 2, by placing PBS as a negative control, IL-8 as a positive control, and ecalectin as a subject compound onto the lower layer, and eosinophils onto the upper layer, a chemotaxis test was conducted using neutrophils. Figure 6 shows the result. A vertical axis shows the number of the stained neutrophils which were passing through pores of the membrane. This result indicates that ecalectin has no or little neutrophil chemotactic activity.

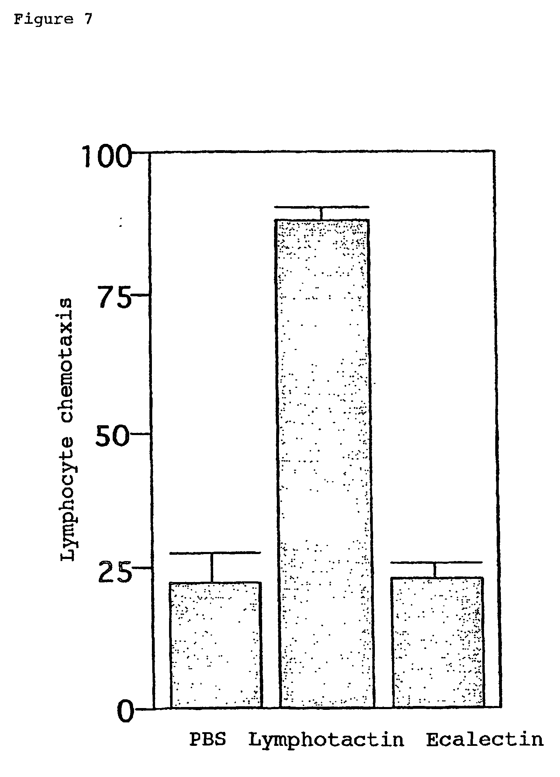

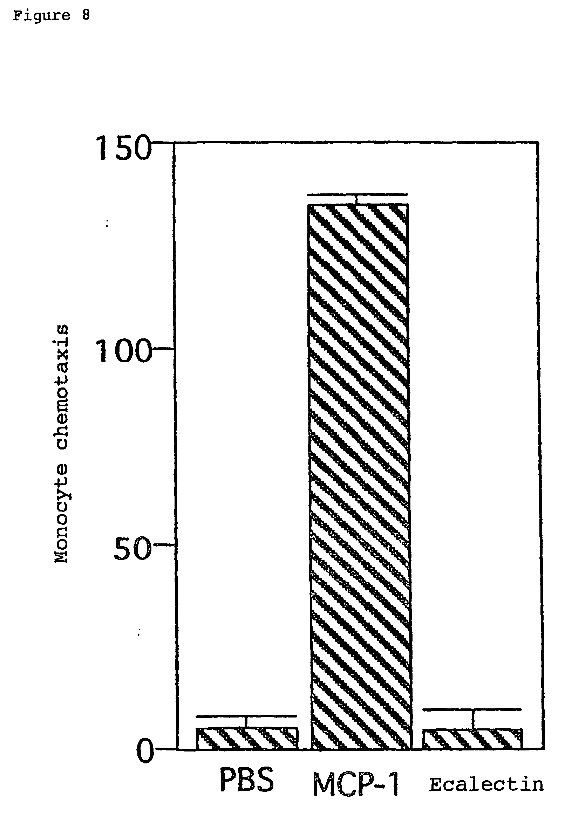

The similar experiments were conducted in regard to lymphocytes (Figure 7) and monocytes (Figure 8). Pore sizes of the membranes are 3 and 8 µm, respectively. In both cases, PBS was used as a negative control. As a positive control, lymphotactin and MCP-1 were used, respectively. A vertical axis shows the number of the stained lymphocytes and monocytes which were passing through the pores of the membranes. As a result, ecalectin was found to comprise no chemotactic activity against lymphocytes or monocytes. The results in Figures 5, 6, 7, and 8 show that ecalectin comprises the chemotactic activity specific to eosinophils. The same specificity was observed in ecalectin expressed in COS cells.

### Industrial Applicability

The present invention provides a compound (protein) comprising a chemotactic activity specific to eosinophils. By using the protein and DNA of the present invention, an agent comprising this protein as an effective ingredient showing an effect of increasing eosinophils within a tissue, can be readily provided. Moreover, a method for screening compounds inhibiting an effect of increasing eosinophils in a tissue is provided, and thus, an agent comprising as an effective ingredient a compound inhibiting the effect obtained by the screening can be provided. The agent can be provided as a therapeutic, a preventive, or an ameliorative drug for various allergic diseases which are typical delayed inflammations attributed to eosinophilia in a tissue, for example, bronchial asthma, allergic rhinitis, and atopic dermatitis.

## Claims

1. An agent for increasing eosinophil chemotaxis, said agent comprising a galectin as an effective ingredient.

2. An agent for decreasing eosinophil chemotaxis, said agent comprising, as an effective ingredient, a compound that inhibits the galectin activity to increase eosinophil chemotaxis.

3. The agent of claim 1 or 2, wherein said galectin is ecalectin.

4. A method for determining whether a test compound reduces eosinophil chemotaxis or not, said method comprising monitoring eosinophil chemotaxis induced in the presence of said test compound together with a galectin and eosinophils, compared with that induced in the absence of said test compound.

5. A method for screening for a compound that reduces eosinophil chemotaxis, said method comprising the steps of:
(a) monitoring eosinophil chemotaxis in the presence of a test compound together with a galectin and eosinophils, compared with that induced in the absence of said test compound, and
(b) selecting a compound that reduces the eosinophil chemotaxis obtained in the step (a).

6. The method of claim 4 or 5, wherein said galectin is ecalectin.

7. A kit comprising a galectin, wherein said kit is provided for the screening of claim 5.

8. The kit of claim 7, wherein said kit further comprises eosinophils.
